(19) Europäisches Patentamt

European Patent Office

Office européen des brevets

(11) Veröffentlichungsnummer: **0 313 762 B1**

# (12) EUROPÄISCHE PATENTSCHRIFT

(45) Veröffentlichungstag der Patentschrift: **04.12.91**

(21) Anmeldenummer: **88114034.7**

(22) Anmeldetag: **29.08.88**

(51) Int. Cl.⁵: **A61F 2/34**

(54) Zweiteilige Hüftgelenkspfanne.

(30) Priorität: **28.10.87 CH 4225/87**

(43) Veröffentlichungstag der Anmeldung:
**03.05.89 Patentblatt 89/18**

(45) Bekanntmachung des Hinweises auf die
Patenterteilung:
**04.12.91 Patentblatt 91/49**

(84) Benannte Vertragsstaaten:
**AT DE FR GB IT**

(56) Entgegenhaltungen:
**DE-C- 3 341 723**
**US-A- 4 695 282**

(73) Patentinhaber: **GEBRÜDER SULZER AKTIENGE-
SELLSCHAFT**
**Zürcherstrasse 9**
**CH-8401 Winterthur(CH)**

Patentinhaber: **Protek AG**
**Stadtbachstrasse 64**
**CH-3001 Bern(CH)**

(72) Erfinder: **Müller, Maurice C., Prof. Dr.-med.**
**Melchenbühlweg 9**
**CH-3006 Bern(CH)**
Erfinder: **Frey, Otto**
**Wallrütistrasse 56**
**CH-8400 Winterthur(CH)**
Erfinder: **Willi, Roland**
**Unterdorfstrasse 24**
**CH-8543 Stadel(CH)**

**Beschreibung**

Die Erfindung betrifft eine zweiteilige Hüftgelenkspfanne, bestehend aus einem im Becken mit Hilfe von Knochenschrauben fixierbaren Verankerungskörper in Form einer Halbkugelschale und aus einem die Pfannenschale für die Aufnahme eines Gelenkkopfes enthaltenden Pfannenkörper aus Kunststoff, wobei der Pfannenkörper durch Einpressen in Achsrichtung der Halbkugelschale mittels eines Schnappverschlusses im Verankerungskörper fixierbar ist, wobei weiterhin der Pfannenkörper mit einem zur Rotations-Symmetrieachse der Halbkugelschale konzentrischen, in einer Führung des Verankerungskörpers geführten Zylindernocken versehen ist.

Eine Hüftgelenkspfanne der vorstehend geschilderten Art ist bereits aus der US-PS 4 695 282 bekannt. Ihre Primärverankerung erfolgt durch Einpressen einer Aussenschale, die sich mit ihrer texturierten Oberfläche auf dem Knochengewebe abstützt. Beim Einbringen der Innenschale zentriert sich diese vor dem Betätigen der Schnappverbindung mit einem entsprechend weit vorstehenden Zylindernocken an einer Bohrung in Richtung der Achse der Aussenschale. Da die Innenschale aus Kunststoff besteht, sollte sie sich in radialer Richtung auf möglichst grossen Durchmessern abstützen, um die Flächenpressung aus Verschleissgründen unter normaler Belastung klein zu halten. Bei der Primärverankerung von Hüftgelenkpfannen sind oft als ergänzendes Befestigungselement Knochenschrauben vorgesehen mit denen die Aussenschale zusätzlich im Knochengewebe gesichert wird. Eine wirkungsvolle Zusatzbefestigung sind Knochenschrauben, die in der Nähe vom Grund der Aussenschale angreifen, um diese in ihr Knochenbett hineinzuziehen. Dem steht entgegen, dass die Schraubenköpfe mit dem für die Zentrierung vorstehenden Zylindernocken bei grossen Zylinderdurchmessern interferieren.

Aufgabe der Erfindung ist es, diese Schwierigkeiten auszuräumen. Diese Aufgabe wird erfindungsgemäss dadurch gelöst, dass der Zylindernocken aus einem dünnwandigen, beim Einpressen des Pfannenkörpers örtlich verformbaren, ringförmigen Steg besteht.

Der dünnwandige, ringförmige Steg verformt sich beim Einpressen des Pfannenkörpers in den Verankerungskörper lokal am Ort des vorstehenden Schraubenkopfes, ohne dass dadurch die Funktionsfähigkeit der Zylinderführung unzulässig beeinträchtigt wird.

Zum Erleichtern des Einpressens des Pfannenkörpers in den Verankerungskörper kann ein in die Halbkugelschale eingreifender Zentrierzapfen bestehen bleiben, um den herum der ringförmige Steg zur Aufnahme von radialen Lasten angeordnet ist; besonders vorteilhaft ist schliesslich die erfindungsgemässe Ausbildung des Pfannenkörpers in Verbindung mit einem Verankerungskörper, bei dem die Durchtrittsöffnungen für die Knochenschrauben im Bereich der Hauptbelastungsrichtung zwischen zwei Meridiankreisen der Halbkugelschale konzentriert sind.

Der Pfannenkörper besteht in bekannter Weise aus einem in der Implantat-Technik gebräuchlichen Kunststoff, beispielsweise aus Polyäthylen, während für den Verankerungskörper in erster Linie ein körperfreundliches Metall, beispielsweise Reintitan oder eine Titanlegierung, Verwendung findet.

Im folgenden wird die Erfindung anhand eines Ausführungsbeispiels im Zusammenhang mit der Zeichnung näher erläutert.

Fig. 1    ist eine, teilweise im Schnitt dargestellte Seitenansicht der neuen Hüftgelenkspfanne, während

Fig. 2    als Detail aus Fig. 1 in vergrössertem Massstab eine Ausführungsform eines Schnappverschlusses zeigt.

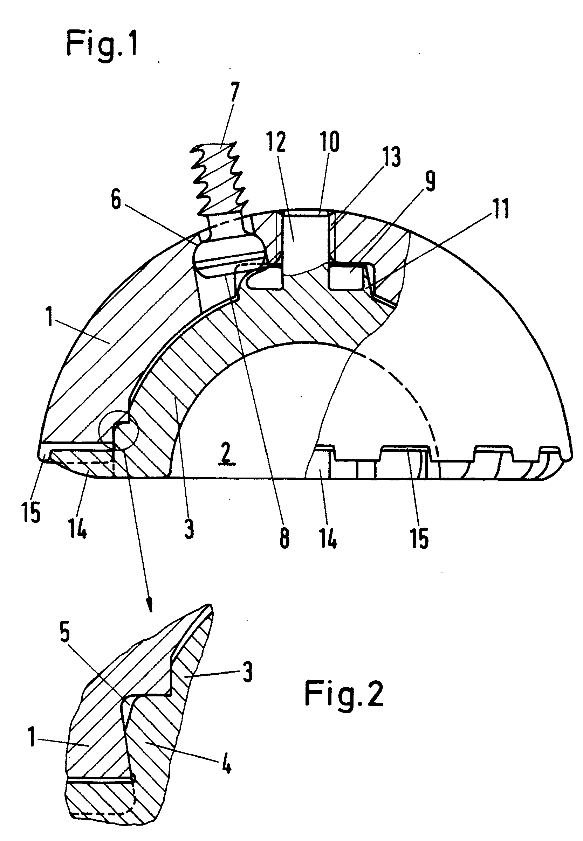

In einem die Form einer Halbkugelschale aufweisenden Verankerungskörper 1 aus Metall ist ein die Pfannenschale 2 für die Aufnahme eines nicht gezeigten Gelenkkopfes enthaltender Pfannenkörper 3 fixiert. Die Fixierung der beiden Teile 1 und 3 erfolgt in bekannter Weise mit Hilfe eines Schnappverschlusses, bei dem ein ringförmiger Ansatz (Fig. 2) auf der Aussenfläche des Pfannenkörpers 3 in eine entsprechende Nut 5 im Innenhohlraum des Verankerungskörpers 1 einschnappt. Wie Fig. 2 zeigt, sind der Ansatz 4 und die Nut 5 mit konischen Flanken versehen, die sich in Einpressrichtung des Pfannenkörpers 3 erweitern.

Für die Primärfixation des Verankerungskörpers 1 sind in diesem Durchtrittsöffnungen 6 für Knochenschrauben 7 vorgesehen, von denen in Fig. 1 nur eine dargestellt ist. Bei Abweichungen der Schraubenachse von der "Radial"-Richtung der Halbkugelschale ragt der Schraubenkopf 8 in eine zylindrische Ausnehmung 9, in der sich der Innenhohlraum des Verankerungskörpers 1 polwärts zum Scheitelbereich hin fortsetzt und die schliesslich in eine zentrale Bohrung 10 übergeht. Die zylindrische Ausnehmung 9 und die Bohrung 10 dienen im Zusammenwirken mit einem Zylindernokken und einem von diesem umschlossenen Zentrierzapfen 12 im Scheitelbereich des Pfannenkörpers 3 zum Zentrieren und Führen des Pfannenkörpers 3 beim Einpressen in den Verankerungskörper 1.

Erfindungsgemäss ist dabei der Zylindernokken als dünnwandiger, beim Einpressen des Pfannenkörpers 3 durch die Schraubenköpfe 8 örtlich verformbarer, ringförmiger Steg 11 ausgebildet.

In die Bohrung 10 des Verankerungskörpers 1 ist darüberhinaus ein Gewinde 13 für eine Verbindung des Verankerungskörpers 1 mit einem nicht dargestellten Setzinstrument eingeschnitten.

Am Aequator ist der Rand des Pfannenkörpers 3 mit einem Kranz radial nach aussen stehender Vorsprünge 14 versehen, die in Nuten 15 in der Aequatorebene des Verankerungskörpers 1 eingreifen und eine intraoperativ einstellbare Verdrehsicherung zwischen den beiden Körpern 1 und 3 bilden. Die Einstellbarkeit wird dabei dadurch erreicht, dass die Vorsprünge 14 und die Nuten 15 in äquidistanten Winkel-"Abständen" auf dem Umfang des Pfannenkörpers 3 bzw. des Verankerungskörpers 1 verteilt sind.

## Patentansprüche

1. Zweiteilige Hüftgelenkspfanne, bestehend aus einem im Becken mit Hilfe von Knochenschrauben fixierbaren Verankerungskörper in Form einer Halbkugelschale und aus einem die Pfannenschale für die Aufnahme eines Gelenkkopfes enthaltenden Pfannenkörper aus Kunststoff, wobei der Pfannenkörper durch Einpressen in Achsrichtung der Halbkugelschale mittels eines Schnappverschlusses im Verankerungskörper fixierbar ist, wobei weiterhin der Pfannenkörper mit einem zur RotationsSymmetrieachse der Halbkugelschale konzentrischen, in einer Führung des Verankerungskörpers geführten Zylindernocken versehen ist, **dadurch gekennzeichnet,** dass der Zylindernocken aus einem dünnwandigen, beim Einpressen des Pfannenkörpers (3) örtlich verformbaren, ringförmigen Steg (11) besteht.

2. Hüftgelenkspfanne nach Anspruch 1, dadurch gekennzeichnet, dass der Pfannenkörper (3) im Scheitelbereich mit einem, in eine zentrale Bohrung (10) der Halbkugelschale eingreifenden, Zentrierzapfen (12) versehen ist, um den herum der ringförmige Steg (11) angeordnet ist.

3. Hüftgelenkspfanne nach Anspruch 1, dadurch gekennzeichnet, dass die Durchtrittsöffnungen (6) für die Knochenschrauben (7) im Verankerungskörper (1) im Bereich der Hauptbelastungsrichtung zwischen zwei Meridiankreisen der Halbkugelschale konzentriert sind.

## Claims

1. A two-piece acetabulum comprising: a fixing member in the form of a hemispherical shell fixable in the pelvis by means of bone screws; and a plastics cup comprising the shell adopted to receive the head of a joint, the cup being fixable in the fixing member by means of a snap fastening by being pressed in axially of the hemispherical shell, the cup also having a cylindrical projection which is concentric of the rotational axis of symmetry of the hemispherical shell and which is guided in a guide in the fixing member, characterised in that the cylindrical projection is in the form of a thin-walled annular web (11) adapted to deform locally when the cup (3) is pressed in.

2. An acetabulum according to claim 1, characterised in that the cup (3) has in its apex zone a centring pin (12) which engages in a central bore (10) in the hemispherical shell and around which the annular web (11) is disposed.

3. An acetabulum according to claim 1, characterised in that the apertures (6) for the bone screws (7) are concentrated in the fixing member (1) near the main loading direction zone between two meridian circles of the hemispherical shell.

## Revendications

1. Cavité cotyloïde en deux parties se composant d'un corps d'ancrage se fixant dans le bassin au moyen de vis à os et ayant la forme d'une coquille hémisphérique et d'un corps de cavité en matière plastique contenant la cavité cotyloïde de logement d'une tête d'articulation, le corps de cavité se fixant dans le corps d'ancrage au moyen d'une fermeture à enclenchement par enfoncement dans la direction de l'axe de la coquille hémisphérique et par ailleurs le corps de cavité comportant un ergot cylindrique concentrique à un axe de symétrie de révolution de la coquille hémisphérique et passant dans un guide du corps d'ancrage, caractérisée en ce que l'ergot cylindrique consiste en une nervure annulaire (11) à paroi mince qui est déformable localement lors de l'enfoncement du corps de cavité (3).

2. Cavité cotyloïde selon la revendication 1, caractérisée en ce que le corps de cavité (3) comprend dans la région du sommet, un tenon de centrage (12) pénétrant dans un trou central (10) de la coquille hémisphérique et autour duquel est disposée la nervure annulaire (11).

3. Cavité cotyloïde selon la revendication 1, caractérisée en ce que les trous de traversée (6) destinés aux vis à os (7) sont concentrés dans le corps d'ancrage (1) dans la région de la direction principale de charge entre deux cercles méridiens de la coquille hémisphérique.

## Fig.1

7
12 10 13 9
6 11
1
2
15 14 3 8 14 15

5
3
1 4

## Fig.2